Europäisches Patentamt

European Patent Office

Office européen des brevets

(19)

(11) Veröffentlichungsnummer: **0 101 603**
**B1**

(12) **EUROPÄISCHE PATENTSCHRIFT**

(45) Veröffentlichungstag der Patentschrift: **09.11.88**

(21) Anmeldenummer: **83108067.6**

(22) Anmeldetag: **16.08.83**

(51) Int. Cl.⁴: **C 07 K 15/14,** C 07 K 15/06,
G 01 N 33/68, A 61 K 37/02

(54) Neues Protein (PP13), Verfahren zu seiner Anreicherung und Gewinnung sowie seine Verwendung.

(30) Priorität: **20.08.82 DE 3230996**

(43) Veröffentlichungstag der Anmeldung:
**29.02.84 Patentblatt 84/09**

(45) Bekanntmachung des Hinweises auf die
Patenterteilung:
**09.11.88 Patentblatt 88/45**

(84) Benannte Vertragsstaaten:
**AT BE CH DE FR GB IT LI LU NL SE**

(56) Entgegenhaltungen:
EP-A-0 009 715
EP-A-0 033 000
EP-A-0 100 522
US-A-2 042 430
CHEMICAL ABSTRACTS, Band 87, Nr. 21, 21.
November 1977, Seiten 167-168,
Zusammenfassung Nr. 163085s, Columbus,
Ohio, US; H. BOHN: "Isolation and
characterization of placental specific proteins
SP1 and PP5"

(73) Patentinhaber: **BEHRINGWERKE
Aktiengesellschaft
Postfach 1140
D-3550 Marburg 1 (DE)**

(72) Erfinder: **Bohn, Hans, Dr.
Oberer Eichweg 26
D-3550 Marburg (DE)**
Erfinder: **Kraus, Walter
Auf der Heide 15
D-3553 Cölbe 3 (DE)**

(74) Vertreter: **Meyer-Dulheuer, Karl-Hermann, Dr.
et al
HOECHST Aktiengesellschaft Zentrale
Patentabteilung Postfach 80 03 20
D-6230 Frankfurt/Main 80 (DE)**

# 0 101 603

**Beschreibung**

Die Erfindung betrifft ein neues Protein (PP13), ein Verfahren zu seiner Anreicherung und Gewinnung aus einem Extrakt menschlicher Plazenten sowie seine Verwendung.

Im Extrakt aus menschlichen Plazenten wurde bereits eine Reihe löslicher Proteine, die aus diesem Gewebe stammen, nachgewiesen (Bohn, H., Placental and Pregnancy Proteins, in Carcino-Embryonic Proteins, Vol. I, Ed., F. G. Lehmann, Elsevier/North-Holland Biomedical Press, 1979).

Die vorliegende Erfindung beschreibt die Isolierung und Charakterisierung eines neuen löslichen Proteins, genannt PP13.

Gegenstand der Erfindung ist das Protein PP13, gekennzeichnet durch
a) eine elektrophoretische Beweglichkeit im Bereich von Albumin,
b) einen isoelektrischen Punkt von 4,75±0,15,
c) einen Sedimentationskoeffizienten $S^0_{20,w}$ von 3,1±0,15,
d) ein in der Ultrazentrifuge bestimmtes Molekulargewicht von 30 000±5 000,
e) ein im Natriumdodecylsulfat (SDS)-haltigen Polyacrylamidgel bestimmtes Molekulargewicht von 29 000±3 000,
f) einen Extinktionskoeffizienten $E^{1\%}_{1\,cm}$ (280 nm) von 9,8±0,3,
g) einen Kohlenhydratanteil von <1% (g/100 g) (Mannose 0,1±0,05%, Galactose 0,2±0,1%, Xylose 0,1±0,05%, Glucosamin 0,2±0,1%, Neuraminsäure nicht nachgewiesen).

Die Aminosäurenzusammensetzung von PP13 ist in der folgenden Tabelle angegeben:

| | Aminosäurenzusammensetzung von PP13 | |
| --- | --- | --- |
| | (Reste pro 100 Reste) Mol % | Variations- koeffizient |
| Lysin | 4,97 | 11,45 |
| Histidin | 2,28 | 31,46 |
| Arginin | 4,38 | 2,87 |
| Asparaginsäure | 12,60 | 4,65 |
| Threonin | 3,94 | 6,05 |
| Serin | 7,83 | 5,96 |
| Glutaminsäure | 10,16 | 2,54 |
| Prolin | 5,53 | 5,53 |
| Glycin | 6,13 | 4,72 |
| Alanin | 3,39 | 2,49 |
| Cystin $\sqrt{2}$ | 3,73 | 2,93 |
| Valin | 9,85 | 6,79 |
| Methionin | 2,09 | 13,3 |
| Isoleucin | 6,11 | 3,79 |
| Leucin | 5,87 | 0,50 |
| Tyrosin | 3,88 | 4,56 |
| Phenylalanin | 5,68 | 6,85 |
| Tryptophan | 1,46 | 3,09 |

Zur Erläuterung der kennzeichnenden Markmale des Gewebsproteins sei folgendes ausgeführt:
Die Untersuchung der elektrophoretischen Beweglichkeit erfolgte in der Mikromodifikation mit dem

2

0 101 603

Gerät Microzone R 200 von Beckman Instruments auf Zelluloseazetatfolien (Firma Sartorius) unter Verwendung von Natriumdiäthylbarbiturat-Puffer, pH 8,6.

Die Bestimmung des isoelektrischen Punktes wurde mit einer Säule (440 ml) der Firma LKB, Stockholm, durchgeführt. Das sogenannte Ampholin(R)-Gemisch hatte bei der Untersuchung des Glykoproteins einen pH-Bereich von 4,0 bis 6,0.

Der Sedimentationskoeffizient wurde in einer analytischen Ultrazentrifuge der Firma Beckman (Spinco-Apparatur, Modell E) bei 60.000 UpM in Doppelsektorzellen mit Hilfe der UV-Scanner Technik bei 280 nm bestimmt. Als Lösungsmittel diente ein 0,05 M Phosphatpuffer (pH 6,8), der 0,2 Mol/l NaCl enthielt. Die Proteinkonzentration wurde auf eine optische Dichte (o.D.) von etwa 3 eingestellt. Der Sedimentationskoeffizient wurde auf die Basis von Wasser bei 20°C umgerechnet.

Zur Ermittlung des Molekulargewichtes in der Ultrazentrifuge wurde die Sedimentationsgleichgewichtsmethode herangezogen. Die Konzentration des Proteins ist dabei auf etwa 1.0 O.D. eingestellt worden. Die Bestimmung wurde bei 9.000 UpM vorgenommen. Die Registrierung erfolgte mit UV-Optik bei 280 nm unter Einsatz des photoelektrischen Scanners.

Zur Bestimmung des Molekulargewichtes im SDS-PAA-Gel wurde ein Gel mit 7,5% (g/100 ml) Polyacrylamid (PAA), das 0,1% (g/100 ml) Natriumdodecylsulfat (SDS) enthielt, verwendet. Als Vergleichssubstanz dienten humanes Plazentalaktogen (HPL) und Human-Albumin sowie dessen Aggregate.

Zur Bestimmung des Extinktionskoeffizienten wurde die Substanz 0,10%ig (g/100 ml) in Aqua dest. gelöst.

Die Analyse der Kohlenhydrate wurde wie folgt durchgeführt: Nach Hydrolyse der glykosidischen Bindungen wurden die freigesetzten Neutralzucker als Boratkomplexe über eine Anionenaustauschersäule getrennt (Y. C. Lee et al., Anal. Biochem. *27*, 567 (1969)), im Eluat durch Zumischung von Cu(I)-bicinchoninatreagenz (K. Mopper und M. Gindler, Anal. Biochem. *56*, 440 (1973)) angefärbt und unter Verwendung von Rhamnose als internem Standard quantitativ bestimmt. Die Aminozucker wurden durch ihre Reaktion mit Ninhydrin nachgewiesen und bestimmt. Der Neuraminsäuregehalt ist mit der Methode nach Warren (Methods in Enzymology, Vol. VI, 463—465 (1963)) ermittelt worden.

Die Aminosäureanalyse wurde nach S. Moore, D. H. Spackman, W. H. Stein, Anal. Chem. *30*, S. 1185 (1958), unter Verwendung des Flüssigkeitschromatographen Multichrom B der Firma Beckman durchgeführt. 1/2 Cystin wurde nach Oxydation der Proteine mit Perameisensäure (S. Moore et al., Anal. Chem. *30*, S. 1185 (1958)) und nachfolgender Chromatographie (S. Moore, J. Biol. Chem., *238*, S. 235 (1963)) als Cysteinsäure bestimmt. Der Tryptophangehalt ist mit der direkten photometrischen Bestimmung nach H. Edelhoch, Biochemistry *6*, S. 1948 (1967), ermittelt worden.

$PP_{13}$ hat folgende Eigenschaften, die sich in einem Verfahren zu seiner Isolierung verwenden lassen, indem diesen Eigenschaften entsprechende Maßnahmen getroffen werden:

1) Mit Ammoniumsulfat wird es bei pH 7,0 und 30—60% Sättigung aus wässrigen Lösungen gefällt.

2) Mit wasserlöslichen Acridinbasen, zum Beispiel 2-Äthoxi-6,9-diaminoacridinlactat (Rivanol®) wird es bei pH-Werten zwischen 4—9 und einer Konzentration der Base von 0,2 bis 0,8% (g/100 ml) präzipitiert. Bei einem pH-Wert von 6,0 und einer Rivanolkonzentration von 0,4% (g/100 ml) fällt es bereits zum Teil aus.

3) Bei der elektrophoretischen Auftrennung wandert es bei pH 8,0 ähnlich schnell wie Albumin.

4) Bei der isoelektrischen Fokussierung erscheint es im pH-Bereich von 4,6 bis 4,9 mit Maximum zwischen 4,7 und 4,8.

5) Bei der Gelfiltration mit Sephadex® verhält es sich wie Proteine mit Molekulargewichten von 10 000 bis 40 000.

6) Es läßt sich an schwach basische Ionenaustauscher wie beispielsweise DEAE-Cellulose oder DEAE-Sephadex bei einer Leitfähigkeit von etwa 0—2 mS und einem pH-Wert von etwa pH 7 bis 9 binden und wird erst unter Verwendung stärker konzentrierter Salzlösungen (1—5 g/100 ml NaCl-Lösungen) vom Ionenaustauscher wieder eluiert.

7) Es kann aus einer wässrigen Lösung durch Immundadsorption angereichert und isoliert werden.

Gegenstand der Erfindung ist ferner ein Verfahren zur Gewinnung des $PP_{13}$, dadurch gekennzeichnet, daß ein Extrakt aus menschlichen Plazenten, unter Verwendung der obenstehenden Eigenschaften fraktioniert wird.

Neben Ammoniumsulfat können selbstverständlich auch andere in der präparativen Biochemie üblicherweise eingesetzte Neutralsalze zur Ausfällung des $PP_{13}$ verwendet werden. Neben einer Acridinbase ist auch ein wasserlösliches Derivat einer Chinolinbase, wie sie für Proteinfraktionierungen bekannt sind, im Rahmen des erfindungsgemäßen Verfahrens einsetzbar. Einsprechend seinem elektrophoretischen Verhalten, seinem isoelektrischen Punkt, seinem Molekulargewicht, sind zur Isolierung des Proteins auch andere Maßnahmen brauchbar, die geeignet sind, ein Protein mit den angegebenen Eigenschaften von anderen Proteinen abzutrennen. Hierzu können die verschiedenen Methoden der präparativen Elektrophorese, der isoelektrischen Fokussierung, der Gelfiltration, Gelchromatographie oder Ultrafiltration oder auch die Eigenschaft des $PP_{13}$, an schwach basische Ionenaustauscher gebunden und hiervon wieder eluiert werden zu können, verwendet werden.

Durch eine zweckmäßige Kombination de genannten Maßnahmen, die eine Anreicherung des $PP_{13}$ oder eine Abtrennung dieses Proteins von anderen Proteinen bewirken, kann das $PP_{13}$ isoliert werden.

Die im Beispiel angegebenen Schritte zur Anreicherung und Isolierung von $PP_{13}$ sind keinesfalls alle zwingend und müssen auch nicht in der dort beschriebenen Reihenfolge durchgeführt werden.

3

Zur Immunoadsorption könnte man direkt den Extrakt aus menschlichen Plazenten einsetzen. Da die Konzentration von $PP_{13}$ im Plazentenextrakt aber relativ gering ist, ist es zweckmäßig, durch eine Vorfraktionierung des Extraktes das Protein $PP_{13}$ erst spezifisch anzureichern mit Hilfe von Methoden, die sich zur Fraktionierung von Proteinen in größerem Maßstab eignen; zum Beispiel durch fraktionierte Fällung mit Neutralsalzen oder organischen Kationen, durch Gelfiltration oder durch Ionenaustauscher-chromatographie. Auch der Schritt der Immunadsorption könnte durch Anwendung anderer Trennmethoden, zum Beispiel durch präparative Elektrophorese und isoelektrische Fokussierung, ersetzt werden.

Zur Hochreinigung von $PP_{13}$ im Endstadium der Isolierung haben sich Gelfiltration an Sephadex G-100 und inverse Immunadsorption als brauchbar erwiesen.

Aus einer ausgewachsenen menschlichen Plazenta (600 g) lassen sich mit physiologischer Salzlösung im Durchschnitt 3,7 mg dieses Proteins extrahieren. Extrakte anderer Organe des Menschen (zum Beispiel aus Herz, Lunge, Haut, Magen, Niere, Uterus, Leber, Milz, Nebenniere, Colon und Blase) enthalten dieses Protein nicht oder in wesentlich geringerer Konzentration. Auch im Serum von anderen Körperflüssigkeiten des Menschen kommt $PP_{13}$ normalerweise nicht oder nur in Spuren (<1 mg/l) vor.

Zum Nachweis und zur Bestimmung des $PP_{13}$ etwa in einer Fraktion aus einer Trennoperation können neben den angegebenen Parametern auch immunchemische Methoden dienen, da $PP_{13}$ antigene Eigenschaften hat. Bei der Immunisierung von Tieren mit diesem Protein werden spezifische Antikörper gebildet.

Ein für diesen Zweck brauchbares Antiserum kann folgendermaßen gewonnen werden: Durch Immunisieren von Kaninchen mit einer $PP_{13}$-enthaltenden Plazentaproteinfraktion (zum Beispiel einer Mutterlauge aus der Kristallisation von humanem Placentalactogen (HPL) nach Bohn H., Experientia *27* (1971) 1223) wird ein polyvalentes Antiserum erhalten, das unter anderem auch Antikörper gegen $PP_{13}$ enthält. Dieses Antiserum kann durch Absorption mit normalem menschlichen Serum und solchen Plazentafraktionen, die $PP_{13}$ nicht enthalten, beziehungsweise mit gereinigten Plazentaproteinen (zum Beispiel HPL, $PP_{11}$, $PP_{12}$ und $PP_{16}$) gegen das Antigen $PP_{13}$ weitgehend spezifisch gemacht werden.

Dieses Antiserum kann einerseits zum immunologischen Nachweis des $PP_{13}$, andererseits zur Herstellung eines Immunadsorbens dienen, das zur Anreicherung und Isolierung von $PP_{13}$ eingesetzt werden kann.

Mit Hilfe des nach der vorliegenden Anmeldung gewonnenen gereinigten $PP_{13}$ lassen sich durch Immunisieren von Tieren nach bekannten Methoden monospezifische Antiseren herstellen.

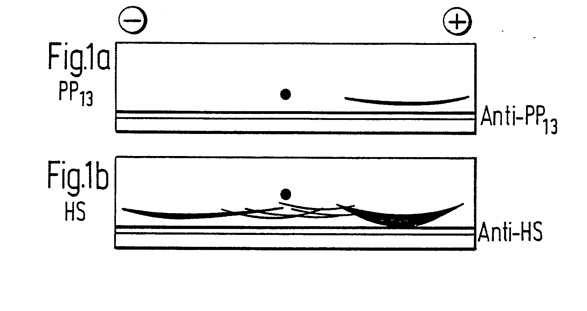

Abbildung 1a zeigt die immunologische Reaktion von $PP_{13}$ mit einem spezifischen Antiserum vom Kaninchen nach Auftrennung im elektrischen Feld in Agar-haltigem Gel.

Abbildung 1b bringt zum Vergleich dazu die Auftrennung der Proteine des Serums, sichtbar gemacht durch deren Immunreaktion mit einem Antiserum vom Kaninchen gegen Humanserum (HS).

Zum immunologischen Nachweis von $PP_{13}$ kann auch die Geldiffusionstechnik nach Ouchterlony (vgl. Schultze and Heremans, Molecular Biology of Human Proteins, Vol. 1, pg. 134) oder wenn notwendig auch empfindlichere Methoden wie Radioimmunoassays oder Enzymimmunoassays herangezogen werden.

Der Nachweis und die Bestimmung von $PP_{13}$ haben diagnostische Bedeutung: $PP_{13}$ ist ein plazenta-"spezifisches" Protein. Solche Proteine sind im allgemeinen mit fortschreitender Schwangerschaft in zunehmendem Maße im Serum erhöht; sie lassen sich daher als Parameter zur Überwachung der Schwangerschaft verwenden. Plazentaspezifische Proteine sind andererseits aber auch vielfach bei Patienten mit Tumoren-insbesondere bei trophoblastischen und embryonalen Tumoren- im Serum nachweisbar beziehungsweise im Gewebe der Tumoren lokalisiert; sie können daher bei solchen Erkrankungen als Marker zur Überwachung des Krankheitsverlaufs und zur Kontrolle der Therapie eingesetzt werden.

$PP_{13}$ kann also verwendet werden, um Antiseren herzustellen, die dazu dienen können, $PP_{13}$ nachzuweisen und zu bestimmen.

Die Erfindung wird am nachstehenden Beispiel erläutert:

Beispiel
A) Extraktion der Plazenten und Fraktionierung des Extraktes mit Rivanol und Ammoniumsulfat

1.000 kg tiefgefrorene menschliche Plazenten wurden im Schneidmischer zerkleinert und mit 1.000 Liter einer 0,4 %igen (g/100 ml) Kochsalzlösung extrahiert. Der Extrakt wurde nach Abtrennung des Geweberückstandes durch Zentrifugation mit 20 %iger (g/100 ml) Essigsäure auf pH 6,0 eingestellt und unter Rühren mit 200 Liter einer 3 %igen (g/100 ml) Lösung von 2-Äthoxy-6,9-Diaminoacridin-Lactat (Rivanol®, Hoechst, AG) versetzt. Der Niederschlag wurde durch Zentrifugation abgetrennt, mit 500 Liter einer 2,5 %igen (g/100 ml) NaCl-Lösung versetzt und 4 Stunden gerührt. Das abgeschiedene Chlorid des 2-Äthoxy-6,9-Diaminoacridins wurde abzentrifugiert. Der Überstand ist unter Rühren langsam mit soviel festem Ammoniumsulfat versetzt worden, daß eine Endkonzentration von 30 % (g/100 ml) erreicht wurde. Der Niederschlag wurde abzentrifugiert. Es wurden dabei 4,5 kg einer feuchten Paste, die im Nachfolgenden als Fraktion A bezeichnet wird, erhalten.

B) Gelfiltration an Sephadex G-150
1.200 g der Fraktion A wurden in Wasser gelöst und gegen einen 0,01 M Tris-HCl-Puffer (pH 8,0), der 0,05% (g/100 ml) NaN$_3$ enthielt (Pufferlösung I), dialysiert. Die zurückbleibende Lösung wurde auf eine mit Sephadex G-150 gefüllte Säule (60×65 cm) aufgetragen und mit Pufferlösung I eluiert. Die Eluate mit den niedermolekularen Proteinen (MG 10 000—40 000) wurden vereinigt und als Fraktion B bezeichnet.

C) Chromatographie an DEAE-Zellulose

Die Proteine der Fraktion B wurden an DEAE-Cellulose (Säule 10×28 cm) adsorbiert. Die Säule ist dann mit Pufferlösung I gespült und mit 5% (g/100 ml) Kochsalzlösung eluiert worden. Das Eluat wurde auf einem Ultrafilter eingeengt und gegen einen 0,1 M Tris-HCl-Puffer von pH 8, der 1 Mol/l NaCl und 0,1% Natriumacid enthielt (Pufferlösung II), dialysiert (Fraktion C).

D) Anreicherung von PP$_{13}$ durch Immunadsorption
1. Herstellung des Immunadsorbens

400 ml eines Anti-PP$_{13}$-Serums vom Kaninchen wurden gegen 0,02 M Phosphatpuffer (pH 7,0) dialysiert und zur Abtrennung der Immunglobuline an DEAE-Cellulose chromatographiert. Die Immunglobulinfraktion (4,69 g Protein) wurde dann mit 469 g besonders gereinigter Agarose in Kugelform (Sepharose® der Pharmacia, Uppsala, Schweden), die mit 58,6 g Bromcyan aktiviert worden war, umgesetzt und so kovalent an einen Träger gebunden. Das Verfahren wurde beschrieben von Axen R., Porath J., Ernbach S., Nature *214*, 1302 (1967). Mit Hilfe eines auf diese Weise hergestellten Immunoadsorbens konnte PP$_{13}$ aus seinen Lösungen, insbesondere aus PP$_{13}$ angereicherten Plazenta-extraktfraktionen isoliert werden.

2. Immunadsorption

Das Immunadsorbens wurde in Pufferlösung II suspendiert, in eine Chromatographiesäule gefüllt (5,5×22 cm) und mit Pufferlösung II nachgespült.

Dann wurde Fraktion C auf die Säule aufgetragen, wobei PP$_{13}$ immunadsorptiv gebunden wurde. Die Säule wurde dann gründlich mit Puffer II gespült, anschließend ist das adsorbierte Protein mit etwa 600 ml 3 M Kaliumrhodanid-Lösung von der Säule eluiert worden. Die PP$_{13}$-haltigen Eluate wurden gegen Pufferlösung II dialysiert und mit einem Ultrafilter auf etwa 20 ml eingeengt. Ausbeute pro Adsorption ~10 mg PP$_{13}$. Das Adsorbens in der Säule wurde unmittelbar nach der Elution von PP$_{13}$ wieder mit Pufferlösung II neutralisiert und gründlich gewaschen; es ist dann erneut zur immunadsorptiven Bindung von PP$_{13}$ eingesetzt worden.

E) Hochreinigung von PP$_{13}$

Das durch Immunadsorption gewonnene Protein war häufig durch unspezifisch gebundene Serumproteine und andere Plazenta-Gewebsproteine verunreinigt. Die Abtrennung der Hauptmenge der Serum-Begleitproteine gelang durch Gelfiltration an Sephadex G-100. Die restlichen Begleitproteine wurden dann durch inverse oder negative Immunadsorption, das heißt mit Hilfe von trägergebundenen Antikörpern gegen die noch als Verunreinigung vorliegenden Protein (HPL, PP$_{12}$, PP$_{16}$ und Serumprotein) entfernt.

**Patentansprüche**

1. Protein PP$_{13}$, gekennzeichnet durch
a) eine elektrophoretische Beweglichkeit im Bereich von Albumin,
b) einen isoelektrischen Punkt von 4,75±0,15,
c) einen Sedimentationskoeffizienten $S^0_{20,w}$ von 3,1±0,15 S,
d) ein in der Ultrazentrifuge bestimmtes Molekulargewicht von 30 000±5 000,
e) ein im Natriumdodecylsulfat (SDS)-haltigen Polyacrylamidgel bestimmtes Molekulargewicht von 29 000±3 000,
f) einen Extinktionskoeffizienten $E^{1\%}_{1cm}$ (280 nm) von 9,8±0,3 und
g) einen Kohlenhydratanteil von <1% (0,1±0,05% Mannose, 0,2±0,1% Galactose, 0,1±0,05% Xylose, 0,2±0,1% Glucosamin, Neuraminsäure nicht nachgewiesen).
2. Verfahren zur Gewinnung des Proteins nach Anspruch 1, dadurch gekennzeichnet, daß ein Extrakt aus menschlichen Plazenten, der ein Protein mit den im Anspruch 1 angegebenen Parametern enthält, einer geeigneten Kombination von mehreren zur Isolierung von Proteinen bekannten Verfahrensmaßnahmen unterworfen wird, wobei jeweils das Material gewonnen wird, welches das Protein mit den angegebenen Eigenschaften enthält, und das Protein gewonnen wird.
3. Verwendung des Proteins nach Anspruch 1 zur Herstellung spezifischer Antikörper gegen diese Protein.
4. Verwendung des Proteins nach Anspruch 1 in immunchemischen Methoden zum Nachweis und zur Bestimmung dieses Proteins.

**Revendications**

1. Protéine PP$_{13}$, caractérisée par

a) une mobilité électrophorétique dans le domaine de l'albumine,

b) un point isoélectrique de 4,75±0,15,

c) un coefficient de sédimentation $S_{20,w}$ de 3,1±0,15 S,

d) une masse moléculaire, déterminée à l'ultracentrifugeuse, de 30 000±5 000,

e) une masse moléculaire déterminée dans un gel de polyacrylamide contenant du dodécylsulfate de sodium (SDS) de 29 000±3 000,

f) un coefficient d'extinction $E_1^{1\%}{}_{cm}$ (280 nm) de 9,8±0,3, et

g) une teneur en hydrates de carbone inférieure à 1% (0,1±0,05% de mannose, 0,2±0,1% de galactose, 0,1±0,05% de xylose, 0,2±0,1% de glucosamine, acide neuraminique non détecté).

2. Procédé de préparation de la protéine suivant la revendication 1, caractérisé en ce qu'on soumet un extrait de placentas humains qui contient une protéine ayant les paramètres indiqués dans la revendication 1, à une combinaison appropriée de plusieurs mesures opératoires connues pour l'isolement des protéines, en obtenant à chaque fois la matière qui contient la protéine présentant les propriétés indiquées, et en obtenant la protéine.

3. Utilisation de la protéine suivant la revendication 1 pour la préparation d'anticorps spécifiques contre cette protéine.

4. Utilisation de la protéine suivant la revendication 1 dans des méthodes immunochimiques de détection et de dosage de cette protéine.

**Claims**

1. The protein PP$_{13}$ which is characterized by

a) an electrophoretic mobility in the same range as albumin,

b) an isoelectric point of 4.75±0.15,

c) a sedimentation coefficient $S_{20,w}^0$ of 3.1±0.1 S,

d) a molecular weight determined by ultracentrifugation of 30,000±5,000,

e) a molecular weight determined in a polyacrylamide gel containing sodium dodecyl sulfate (SDS) of 29,000±3,000,

f) an extinction coefficient $E_1^{1\%}{}_{cm}$ (280 nm) of 9.8±0.3 and

g) a carbohydrate content of <1% (0.1±0.05% mannose, 0.2±0.1% galactose, 0.1±0.05% xylose, 0.2±0.1% glucosamine, and neuraminic acid not detected).

2. A process for isolating the protein as claimed in claim 1, which comprises subjecting an extract of human placentae which contains a protein having the parameters indicated in claim 1 to a suitable combination of several known procedures for the isolation of proteins, in each case that material which contains the protein having the indicated properties being isolated, and the protein being isolated.

3. The use of the protein as claimed in claim 1 for the preparation of specific antibodies against this protein.

4. The use of the protein as claimed in claim 1 in immunochemical methods for the detection and determination of this protein.

6

Fig.1a
PP₁₃

Anti-PP₁₃

Fig.1b
HS

Anti-HS